# EUROPEAN PATENT SPECIFICATION

(11) **EP 0 379 139 B1**
(45) Date of publication and mention of the grant of the patent: **27.07.1994**
(21) Application number: 90100814.4
(22) Date of filing: 16.01.1990
(51) Int. Cl.: A61B 8/06, G01S 15/89, G01S 15/58, G01S 7/52

(54) **Ultrasonic diagnostic apparatus**
Ultraschalldiagnosegerät
Appareil de diagnostic ultrasonore

(30) Priority: 17.01.1989 JP 8065/89
(43) Date of publication of application: 25.07.1990
(73) Proprietor: FUJITSU LIMITED, Kawasaki-shi, Kanagawa 211 (JP)
(72) Inventor: Amemiya, Shinichi, c/o Fujitsu Ltd., Pat. Dep., Kawasaki-shi, Kanagawa 211 (JP)
(74) Representative: Sunderland, James Harry

(56) References cited:
- EP-A- 0 011 878
- EP-A- 0 235 822
- WO-A-85/02105
- DE-A- 3 543 604
- US-A- 4 324 258

## Description

The present invention relates to ultrasonic diagnostic apparatus for displaying a moving body in colour, and is concerned particularly but not exclusively with ultrasonic analysis equipment that provides frequency and sign outputs corresponding to flow speed and flow direction of blood flow by means of a colour flow mapping analyser.

An ultrasonic diagnostic apparatus has been proposed which utilises a colour flow mapping analyser, applies ultrasonic waves to a human body and measures frequency difference between the applied and reflected waves to determine speed of blood flow. Thus, the ultrasonic diagnostic apparatus offers the promise of easy measurement of cardiac blood flow.

It has been proposed that a colour flow mapping analyser incorporated in such ultrasonic diagnostic apparatus orthogonally detect a received signal determined from a reflected wave, using a sent signal corresponding to an applied wave and a signal whose phase is offset from the sent signal by 90 degrees to obtain two output signals, X and Y, whose phases are offset by 90 degrees from each other. After the removal of unnecessary signals such as those from the heart wall, an autocorrelation coefficient is determined from the two output signals, X and Y. An absolute frequency value from the determined autocorrelation coefficient is output, with f, s and p denoting frequency, the positive or negative sign of the frequency from the autocorrelation coefficient, and the strength of the two output signals, X and Y, respectively. Thus, frequency f output from the colour flow mapping analyser reflects the speed of blood flow, sign s indicates its direction and strength p represents the strength of the reflected wave.

In a proposed ultrasonic diagnostic apparatus utilising a colour flow mapping analyser, a positive sign s output therefrom indicates the shade, corresponding to frequency f, of a colour, e.g. blue, and a negative sign s indicates the shade, corresponding to frequency f, of another colour, e.g. red, to provide images covering the distribution of blood flow. If strength p output from the colour flow mapping analyser is below a given threshold, the value of frequency f is replaced by 0, since the SN ratio of the reflected wave is degraded. Thus, the effects of noise can be eliminated to some extent.

A prior art example ultrasonic diagnostic apparatus is disclosed in EP-A-235 822 (see also Japanese Patent Publication (Laid-open) No. 270139-1987).

This prior art is such that, if strength p output from the colour flow mapping analyser is below a given threshold, frequency f is replaced by 0. Since the setting of too high a threshold results in degraded image quality (black holes appear in the image), however, a threshold is set within allowable measuring accuracy. Nonetheless, frequency f output from the colour flow mapping analyser corresponding to strength near a threshold contains slight noise, making it impossible to completely eliminate the effects of noise.

Contained noise leads to random values of frequency f and sign s, causing partial inversion of values of sign s. A result is the occurrence of a problem in that the blue colour indicating that blood flows in a certain direction is mixed with red colour (indicating an opposite or reverse flow) and images are disordered.

Such disorder in images might conceivably result in improper diagnosis in hospitals which use this prior art ultrasonic diagnostic apparatus.

A possible method of solving the problem of disorder of images caused by the inversion of sign s, by noise, is such that signs s arising in correspondence to a given area are averaged, to compensate for local variations in sign s. Although, in general, low-speed blood flow encounters or involves no reverse flows, it is in fact observed that high-speed blood flow, classified as turbulent flow, involves local reverse flows. Thus, the compensation intended to improve image quality by averaging signs s also has the effect of making uniform local reverse flow caused by turbulent flow. Hence, such compensation cannot be applied in reality.

US-A-4 324 258 is concerned with an ultrasonic Doppler flowmeter which is operable to provide flow analysis factors, comprising a sign s corresponding to the direction of movement of a moving body and a frequency f corresponding to the speed of movement of the moving body, on the basis of received ultrasonic signals reflected from the moving body, and is operable to display the speed distribution of the moving body on the basis the frequency f, or a quantity derived from the frequency f. Zero crossings of filtered and digitized Doppler information are used to determine Doppler frequency. Digital counts representative of zero crossings are accumulated and compared against a predetermined frequency (the Nyquist criterion): if the counts exceed the predetermined value the sign as determined just prior to the value being exceeded is used in preference to the instantaneous sign.

According to the present invention there is provided ultrasonic diagnostic apparatus, comprising:
a colour flow mapping analyser operable to provide flow analysis factors, comprising a sign s corresponding to the direction of movement of a moving body and a frequency f corresponding to the speed of movement of the moving body, on the basis of received ultrasonic signals reflected from the moving body,
comparator circuitry operable to compare in magnitude one or more flow analysis factors, or quantities derived from the factor or those factors concerned, with a threshold or with respective thresholds, and
means for displaying the speed distribution of the moving body on the basis the frequency f, or a quantity derived from the frequency f,
characterised in that
the apparatus further comprises:-
an average sign circuit operable to calculate in respect of a given period the average s̅ of signs s provided by the colour flow mapping analyser, and
sign selection circuitry arranged to receive the results of comparisons effected in the comparator circuitry and to select either sign s or the average sign s̅ calculated by the average sign circuit for output, in dependence upon the said results,
and in that
the means for displaying the speed distribution of the moving body do so on the basis of the output of the sign selection circuitry and the frequency f, or a quantity derived from the frequency f.

An embodiment of the present invention can provide for images which do not suffer, or suffer to a lesser degree, disorder caused by noise.

An embodiment of the present invention can provide for a reduction in the possibility of false diagnosis originating from imaging problems.

In an embodiment of the present invention at least one of frequency and strength comparators is provided and a selection effected by a sign selection circuit is controlled according to the output of the comparator(s). The sign selection circuit receives the signs output from the colour flow mapping analyser and the average thereof, selecting and outputting said signs or the average. Selection conditions employed can be, for example,
(i) the frequency analysed by the colour flow mapping analyser is below a certain value, or
(ii) the strength analysed by the colour flow mapping analyser is below a certain value, or
(iii) both of conditions (i) and (ii) are met, or
(iv) at least one of conditions (i) and (ii) is met. In each case when the selection conditions are satisfied
the average of signs is selected whilst in other cases a sign itself is selected as an analysis result.

Reference is made, by way of example, to the accompanying drawings, in which:-
Fig. 1 illustrates the configuration of ultrasonic diagnostic apparatus in accordance with an embodiment of the present invention;
Fig. 2 illustrates a detailed configuration of a colour flow mapping analyser which can be employed in the embodiment of Fig. 1;
Fig. 3 illustrates a detailed configuration of a noise filtering circuit which can be employed in the embodiment of Fig. 1;
Fig. 4 illustrates a detailed configuration of a sign averaging circuit which can be employed in the embodiment of Fig. 1;
Fig. 5 illustrates a detailed configuration of an equivalent delaying circuit which can be employed in the embodiment of Fig. 1;
Fig. 6 illustrates a detailed configuration of a frequency averaging circuit which can be employed in the embodiment of Fig. 1;
Fig. 7 illustrates a detailed configuration of a mode control circuit which can be employed in the embodiment of Fig. 1;
Fig. 8 illustrates a detailed configuration of a sample sign selection circuit and a sample selection circuit of a mode control circuit which can be employed in the embodiment of Fig. 1;
Fig. 9 illustrates an alternative configuration which can be employed in ultrasonic diagnostic apparatus embodying the present invention; and
Fig. 10 illustrates another alternative configuration which can be employed in ultrasonic diagnostic apparatus embodying the present invention.

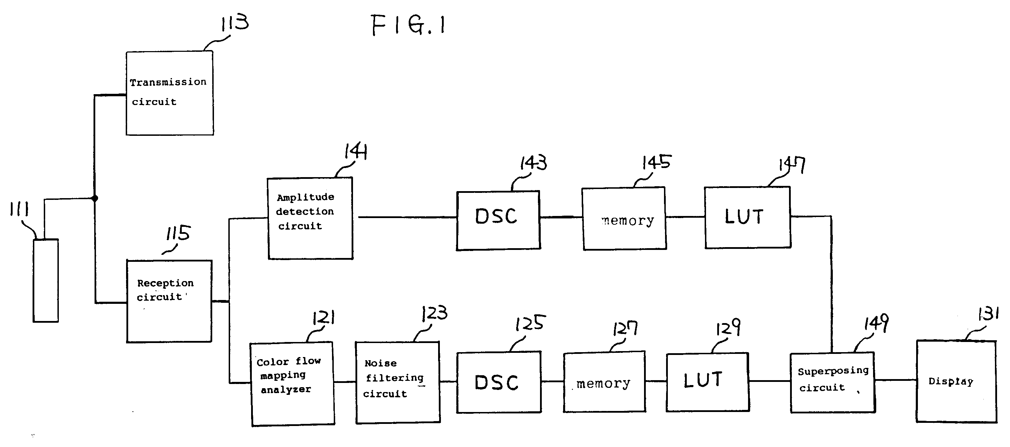

In Fig. 1, 111 is an ultrasonic probe, 113 a transmission circuit, 115 a reception circuit, 121 a colour flow mapping analyser, 123 a noise filtering circuit, 125 and 143 digital scanning converters, 127 and 145 memory, 129 and 147 look-up tables, 131 a display, 141 an amplitude detection circuit, and 149 a superposing circuit.

Ultrasonic probe 111 has, in this embodiment, a configuration which is e.g. a phased array, being provided with many (about several billions) ultrasonic elements. Ultrasonic probe 111 is connected to transmission circuit 113 and reception circuit 115.

Transmission circuit 113, which drives ultrasonic probe 111, excites the ultrasonic elements incorporated in the probe to generate ultrasonic waves with a certain irradiation angle. Reception circuit 115, which is used to detect ultrasonic waves reflected by an object to be measured (a moving body) when the reflected waves reach ultrasonic probe 111, delays and adds the signals from ultrasonic elements in order to provide for the detection of the reflected waves.

Colour flow mapping analyser 121 analyses the speed of an object measured in accordance with signals received from reception circuit 115. After this analysis, colour flow mapping analyser 121 generates frequency output f corresponding to the speed of the object measured, sign output s corresponding to the speed direction (direction of movement of the object), and strength output p corresponding to the strength of reflected waves. Output from the colour flow mapping analyser is entered into noise filtering circuit 123.

Noise filtering circuit 123 corrects sign s output from colour flow mapping analyser 121 in accordance with a certain preset mode. This correction is for dealing with inversion of sign by noise. When a state in which the sign may be inverted by noise is detected, mean value s̅ of a required period (taken over a required interval) is output in place of sign s. In such a case, the signs of e.g. 15 successive pixels, before and after one displayed pixel, are averaged to determine the sign (for display) of the one pixel contained in the 15 pixels. Sign s output from noise filtering circuit 123, or its average value s̅ and frequency f are entered into digital scanning converter 125.

Digital scanning converter 125 serves for sequentially scanning and fetching output from noise filtering circuit 123. The fetched data is entered into memory 127. Scanning by digital scanning converter 125 is synchronised with scanning which fetches reflected wave signals from ultrasonic probe 111. Memory 127 sequentially stores data output from digital scanning converter 125 into addresses corresponding to positions of the object measured. The data stored in memory 127 is sequentially read and entered into look-up table 129.

Look-up table 129 creates colour data (e.g. RGB data) on the basis of data read from memory 127. Memory 127 stores frequencies f and signs s (or average values s̅) output from noise filtering circuit 123. The stored data is converted into colour data based on the speed of the object measured. For example, a positive sign s (or average value s̅) corresponds to red, and colour data is obtained by making the shade of the red correspond to frequency f. On the other hand, a negative sign s (or average value s̅) corresponds to blue, and colour data is obtained by making the shade of the blue correspond to frequency f. The obtained colour data is entered into display 131.

Display 131 displays the speed of the object measured in colour on the basis of colour data output from look-up table 129. For example, assuming that the object measured is blood, blood flow in a certain direction is displayed in red (or blue). High-speed flow (turbulent flow) sometimes contains flow direction inversions, being displayed by a mixture of blue and red.

Display 131 is suitable for indicating blood flow. The shape of an object can be measured using reflected ultrasonic waves. The following description relates to such shape measurement.

Amplitude detection circuit 141 detects the amplitude of a signal received from reception circuit 115. Amplitude detection circuit 141 provides an output corresponding to the amplitude of a received signal, i.e. a detected output corresponding to the strength of a reflected wave reaching ultrasonic wave probe 111. The detected output is scanned by digital scanning converter 143, stored into memory 145, and entered into look-up table 147. Look-up table 147 creates monochromatic data (with the same RGB value) using data read from memory 145. For example, applying ultrasonic waves to a cross-section of a heart results in different detected outputs from amplitude detection circuit 141, since the strength of reflected waves is different as between the heart wall and the inner section of the heart through which blood flows. This difference is displayed on display 131 as the shape of an object measured. Superposing circuit 149 superposes output from tables 129 and 147 for supply to display 131.

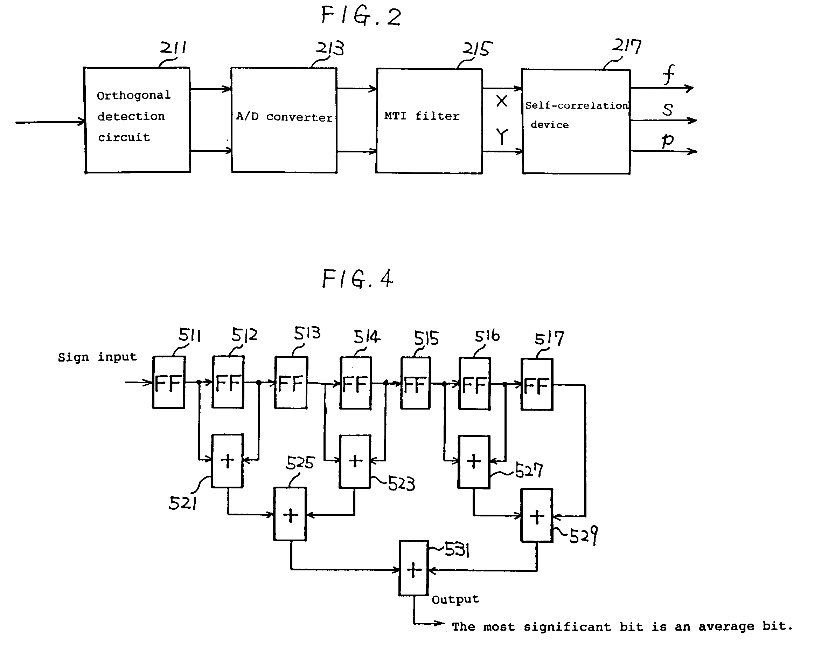

Given below are descriptions of exemplary detailed configurations and operations of features which may be employed in an embodiment of the present invention, referring to Figs. 2 to 10.

Fig. 2 shows a detailed configuration of a colour flow mapping analyser 121. 211 is an orthogonal detection circuit, 213 an analog/digital (A/D) converter, 215 a motion target indicator (MTI) filter, and 217 an autocorrelation device.

Orthogonal detection circuit 211 orthogonally detects received output from reception circuit 115 by means of a signal with the same frequency as output frequency fc of an applied wave and a signal with frequency offset from output frequency fc by 90 degrees. This orthogonal detection provides two output signals whose phases are offset by 90 degrees from each other, which signals are entered into A/D converter 213.

A/D converter 213 converts the two entered signals into corresponding digital data. The thus obtained digital data is entered into MTI filter 215.

MTI filter 215 serves as a high pass filter for two pieces of digital data entered from A/D converter 213. For example, if ultrasonic probe 111 has repeatedly received reflected waves relating to the same position of an object being measured and if MTI filter 215 has subtracted the two waves, MTI filter 215 serves as a one-degree high pass filter for the output data from A/D converter 213 corresponding to the above-reflected waves. Thus, the movement of an object measured leads to higher output values from MTI filter 215, depending on the movement speed. Two pieces of output from MTI filter 215 are entered into autocorrelation device 217.

Autocorrelation device 217 determines a frequency from autocorrelation on the basis of two pieces of output from MTI filter 215. Assuming that the frequency determined from autocorrelation is F and two pieces of output delivered from MTI filter 215 are X(i) and Y(i) (where i refers to the number of transmissions and receptions repeated on the same position of an object measured), the frequency obtained from autocorrelation is:- 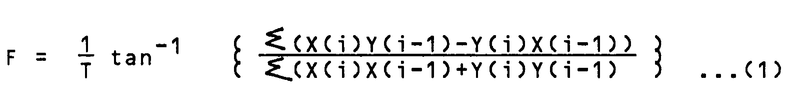
where T indicates the period of repeated transmission or reception repeated on the same position of the object measured.

Autocorrelation device 217 produces absolute value | F | of the frequency determined from autocorrelation according to expression (1), as frequency f, and the sign of autocorrelation coefficient as s. Autocorrelation device 217 produces the total of the square sum of X(i) and Y(i), Σ(X(i)² + Y(i)²), corresponding to the same position of the object measured, as strength p, which expresses the strength of reflected waves. The output from autocorrelation device 217 is entered into noise filtering circuit 123.

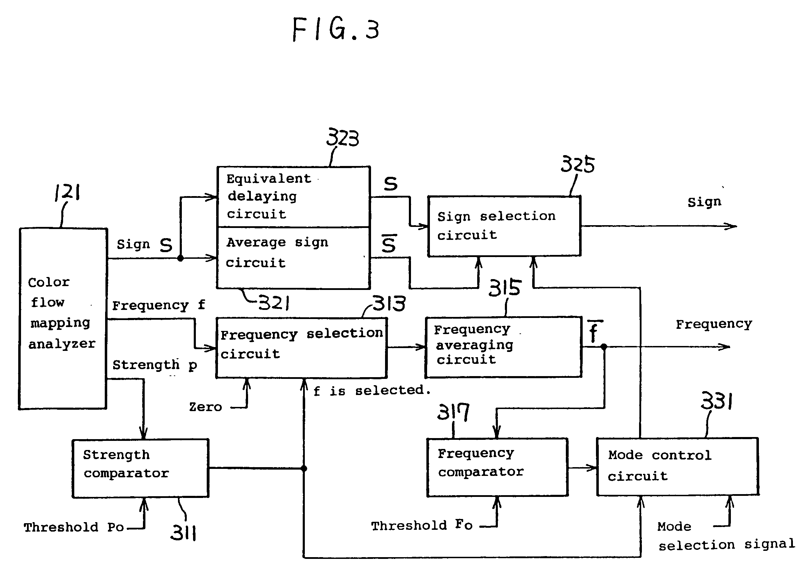

Fig. 3 shows a detailed configuration of a noise filtering circuit 123, where 311 is a strength comparator, 313 a frequency selection circuit, 315 a frequency averaging circuit, 317 a frequency comparator, 321 an average sign circuit, 323 an equivalent delaying circuit, 325 a sign selection circuit, and 331 a mode control circuit.

Strength comparator 311 receives strength p from autocorrelation device 217 in colour flow mapping analyser 121, and compares the strength with a given threshold Po on the basis of their magnitudes. The result of this comparison is entered into frequency selection circuit 313 and mode control circuit 331.

Frequency selection circuit 313 receives frequency f from autocorrelation device 217 in colour flow mapping analyser 121. Frequency selection circuit 313 outputs frequency f as it is or replaces the frequency with 0, depending on the result of comparison by strength comparator 311. More concretely, if strength p entered into strength comparator 311 is below threshold Po, frequency f is affected significantly by noise and, thus, the frequency is replaced with 0 for output. In the other cases, frequency f is output, as it is. The output from frequency selection circuit 313 is entered into frequency averaging circuit 315.

Frequency averaging circuit 315 determines the average value of movement in a given period from the output of frequency selection circuit 313, to provide an average frequency f̅. Determining average frequency f̅ from frequency f makes possible the correction of images by correlation, permitting the provision of an easy-to-see indication on display 131. The output from frequency averaging circuit 315 is entered into frequency comparator 317 and is also output from the noise filtering circuit 123.

Frequency comparator 317 compares the entered average frequency f̅ with a given threshold Fo on the basis of their magnitudes. The result of this comparison is entered into mode control circuit 331.

Mode control circuit 331 controls selection by sign selection circuit 325 in accordance with output from strength comparator 311 and/or output from frequency comparator 317.

Depending on a mode selection signal also entered into mode control circuit 331, the control mode is selected and a control signal conforming to the set control mode is entered into sign selection circuit 325.

Average sign circuit 321 and equivalent delaying circuit 323 receive sign s output from autocorrelation device 217 in colour flow mapping analyser 121. Average sign circuit 321 calculates and outputs an average (s̅) of sign s over a certain period. The average sign covers a range of pixels displayed; e.g., the signs of 7 successive pixels are averaged for output as average sign s̅. Average sign s̅ output from average sign circuit 321 is entered into sign selection circuit 325. Equivalent delaying circuit 323 is for providing synchronisation with the period taken by average sign circuit 321 to calculate the average sign. Entered sign s is delayed over a certain period before being output. Sign s delayed by equivalent delaying circuit is entered into sign selection circuit 325.

Sign selection circuit selects and outputs one of average sign s̅ received from average sign circuit 321 and sign s received from equivalent delaying circuit 323. This selection is controlled by a control signal entered from mode control circuit 331. Sign s or average sign s̅ output from sign selection circuit is output from the noise filtering circuit 123.

Table 1 gives details of exemplary control modes dealt with in mode control circuit 331. A description of these control modes is given below:-

**Table 1**

| | Input signal | Selection |
|---|---|---|
| Mode 1 | f̅ > Fo | s |
| | f̅ ≦ Fo | s̅ |
| Mode 2 | p > Po | s |
| | p ≦ Po | s̅ |
| Mode 3 | Modes 1 and 2 (f̅ > Fo)∩(p > Po) | s |
| | Other cases | s̅ |
| Mode 4 | Mode 1 or 2 (f̅ > Fo)∪(p > Po) | s |
| | Other cases | s̅ |

As is well-known, little reverse flow occurs in blood flowing at low speed. Control mode 1 is intended to take advantage of this fact.

When mode control circuit 331 has received a mode selection signal corresponding to mode 1 it selects average sign s̅ when average frequency f̅ is below (or equal to) a given threshold Fo, and selects sign s in other cases. Since frequency comparator 317 compares average frequency f̅ with given threshold Fo, mode control circuit 331 controls selection in sign selection circuit 325 on the basis of the result of this comparison.

Thus, in mode 1, noise filtering circuit 123 outputs average sign s̅ in place of sign s, if an object measured is moving at low speed, and outputs sign s in the other cases. Little reverse flow exists when low speed movement is involved. This allows replacement (of s) by average sign s̅ without any substantial problems.

When mode control circuit 331 has received a mode selection signal corresponding to mode 2 it selects average sign s̅, when strength p is below (or equivalent to) a given threshold Po, and selects sign s in other cases. Since strength comparator 311 compares strength p with given threshold Po, mode control circuit 331 controls selection by sign selection circuit 325 on the basis of the result of this comparison.

Thus, in mode 2, noise filtering circuit 123 outputs average sign s̅ when few waves are reflected by an object measured and strength p is low, and outputs sign s in other cases. Low-strength p indicates that no significant waves are reflected. This allows replacement by average sign s̅ without any substantial problems.

When mode control circuit 331 has received a mode selection signal corresponding to mode 3 it selects sign s when average frequency f̅ is greater than a given threshold Fo and strength p is greater than a given threshold Po, and selects average sign s̅ in other cases. Mode control circuit 331 then controls selection by sign selection circuit 325 on the basis of the results of comparison in both frequency comparator 317 and strength comparator 325.

When mode control circuit 331 has received a mode selection signal corresponding to mode 4 it selects sign s when either the condition that average frequency f̅ is greater than a given threshold Fo or the condition that strength p greater than a given threshold Po is met, and selects average sign s̅ in other cases (average frequency f̅ is below or equal to given threshold Fo and strength p is below or equal to given threshold Po). Mode control circuit 331 controls selection by sign selection circuit 325 on the basis of the results of comparison by both frequency comparator 317 and strength comparator 311.

Thus, average sign s̅ is selected instead of sign s in mode 1 when an object measured is moving at low speed, in mode 2 when low amplitude waves are reflected, in mode 3 when both of these conditions are met, and in mode 4 when any (either) of these conditions is met. Noise filtering circuit 123 outputs selected sign (s or s̅) and average frequency f̅ and display 131 subsequently uses these outputs for providing its display indication. Based on selected sign (s or s̅), indication by display 131 is clean or cleaner, with noise effects removed or mitigated.

The removal or mitigation from the noise indication provided by display 131 prevents hospital staff from being worried by noise and thus assists in avoiding false diagnosis.

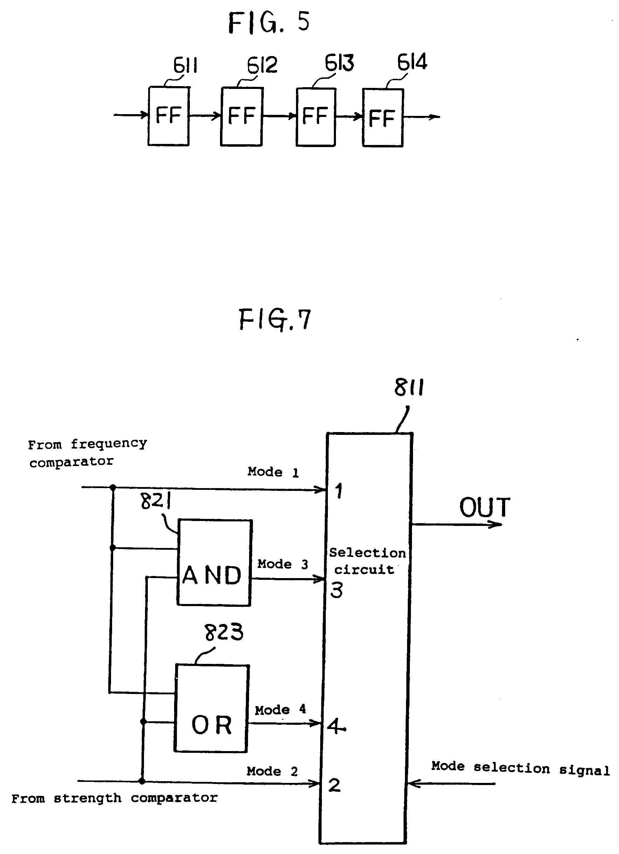

Fig. 4 shows a detailed configuration of an average sign circuit 321. Here, 511, 512, 513, 514, 515, 516 and 517 are flip-flops (FFs), and 521, 523, 525, 527, 529 and 531 are adders.

For example, sign s consists of 1 bit of data, with "1" and "0" corresponding to positive and negative signs, respectively. Average sign circuit 321 averages 7 cycles of signs s. Seven flip-flops 511 to 517 are connected in series.

The output terminals of flip-flops 511 and 512 are connected to the two input terminals of adder 521. The output terminals of flip-flops 513 and 514 are connected to the two input terminals of adder 523. The output terminals of flip-flops 515 and 516 are the two input terminals of adder 527. The output terminal of flip-flop 517 and that of adder 527 are connected to the two input terminals of adder 529. The output terminals of adders 521 and 523 are connected to the two input terminals of adder 525. The output terminals of adders 525 and 529 are connected to the two input terminals of adder 531.

Thus, the sequential entry of signs s into flip-flop 511 leads to the addition by adder 531 of each piece (bit) of the output of the seven serially connected flip-flops, with the most significant bit of the result (consisting of 3 bits) generated as average sign s̅. In fact, if the result of the addition is not less than "100" (4 in decimal number), "1" is output and, if the result is less than "100", "0" is output as average sign s̅.

Fig. 5 shows a detailed configuration of an equivalent delaying circuit 323. Here, 611, 612, 613 and 614 are flip-flops (FFs). Four flip-flops 611 to 614 are connected in series, with sign s entered into flip-flop 611 and output from flip-flop 614, 4 cycles later.

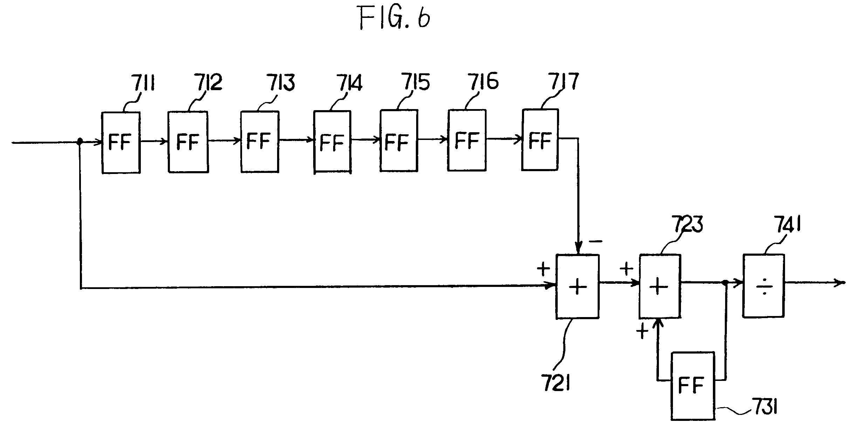

Fig. 6 indicates a detailed configuration employed in a frequency averaging circuit 315. Here, 711, 712, 713, 714, 715, 716, 717 and 731 are flip-flops (FFs), 721 and 723 are adders, and 741 is a divider.

Frequency f received by frequency averaging circuit 315 is entered into input terminals of flip-flop 711 and adder 721. Flip-flops 711 to 717 are connected in series, with the output of flip-flop 717 entered into the other input terminal of adder 721, which is an inverting input terminal. Adder 721 subtracts the output of flip-flop 717 entered into the other input terminal from frequency f entered into the one input terminal.

The output terminal of adder 721 is connected to one input terminal of adder 723, and the output terminal of adder 723 is connected to the input terminal of divider 741 and to the other input terminal of adder 723 via flip-flop 731. The output of adder 723, after it has been stored in flip-flop 731, is entered again into adder 723 itself, resulting in accumulation of the output of adder 721. Such configuration permits the determination of the value of accumulating frequencies f over cycles corresponding to the number of flip-flops 711 to 717.

Divider 741 divides the output of adder 723 by the number of flip-flops 711 to 717 (7), providing average frequency f̅ as the result of this division.

The result of adding each piece (bit) of output from flip-flops 711 to 717 can be divided by a given value to determine average frequency f̅. However, the configuration shown in Fig. 6 permits the number of adders used to be reduced. Setting the number of flip-flops to be connected in series at a multiple of 2 permits the performance of division by divider 741 as bit operation, making it possible to further simplify the configuration.

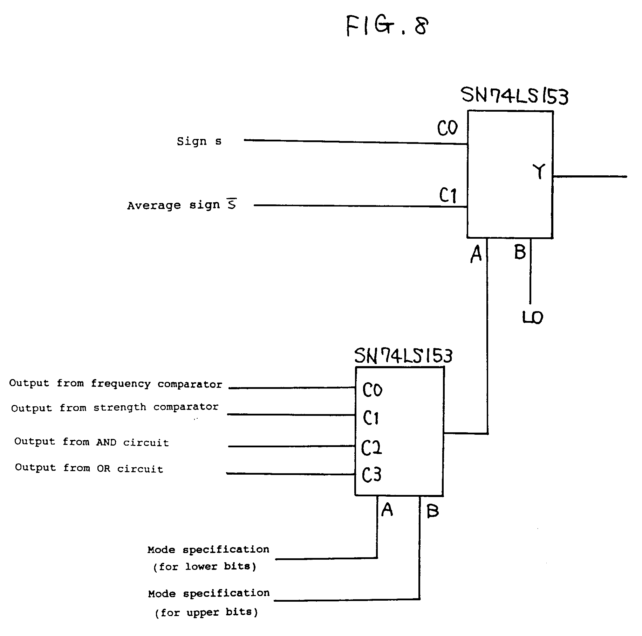

Fig. 7 shows a detailed configuration of a mode control circuit 331, where 811 is a selection circuit, 821 an AND circuit, and 823 an OR circuit.

Frequency comparator 317 is designed to output "1", if frequency f is below (or equal to) a given threshold Fo, and "0" in other cases. Also, strength comparator 311 is designed to output "1", if strength p is below (or equal to) a given threshold Po, and "0" in other cases.

The output of frequency comparator 317 is entered into the first input terminal of selection circuit 811, one input terminal of AND circuit 821, and one input terminal of OR circuit 823. The output of strength comparator 311 is entered into the second input terminal of selection circuit 811, the other input terminal of AND circuit 821, and the other input terminal of OR circuit 823. The output of AND circuit 821 is entered into the third input terminal of selection circuit 811 and that of OR circuit 823 into the fourth input terminal of selection circuit 811.

Selection circuit 811 is provided with a control terminal for receiving mode selection signals in addition to the above four input terminals. Selection circuit 811 selects the signal entered into the first input terminal when a mode selection signal indicating mode 1 is entered into the control terminal. It selects the signal entered into the second input terminal when a mode selection signal indicating mode 2 is entered into the control terminal. It selects the signal entered into the third input terminal when a mode selection signal indicating mode 3 is entered into the control terminal. It selects the signal entered into the fourth input terminal when a mode selection signal indicating mode 4 is entered into the control terminal.

Sign selection circuit 325 provides selection based on output from said selection circuit 811. It selects average sign s̅ output from average sign circuit 321, when selection circuit 811 outputs "1", and sign s output from equivalent delaying circuit 323 in other cases.

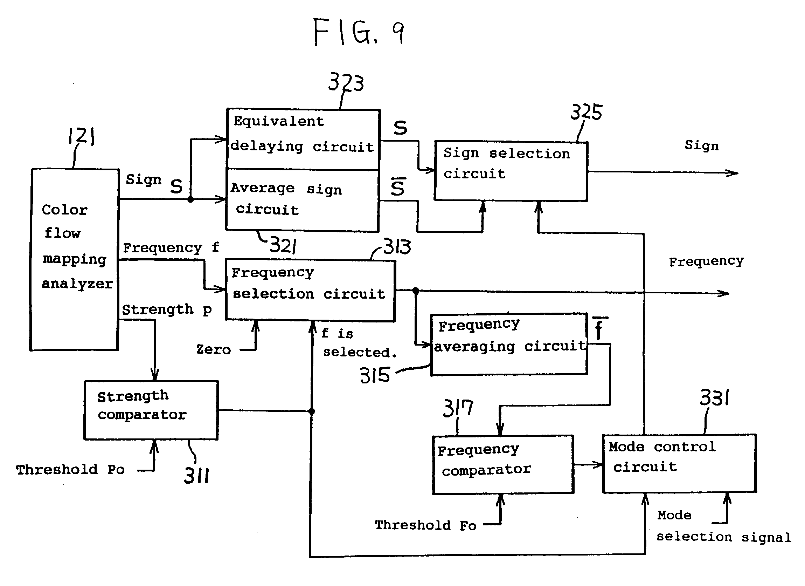

Fig. 8 shows a configuration of a sign selection circuit 325 and selection circuit 811 in mode control circuit 331 implemented using standard logic SN74LS153 (data selector) components manufactured by TI Inc. The standard logic can select one of four input terminals C0 to C3 in accordance with the input logic of input terminals A and B. Four bits "00" to "11" corresponding to modes 1 to 4 are entered into input terminals A and B. Sign selection circuit 325, which covers the above two items of input, uses C0 and C1 of the four input terminals to provide selection in accordance with the input logic of input terminal A (input terminal B continues to receive a fixed low-level signal).

Strength comparator 311 and frequency comparator 317 can be implemented using standard logic components, SN74LS682 to 685 (magnitude comparators), manufactured by TI Inc. In that case, P is an input value of frequency or strength and Q is a set value for comparison (threshold Po or Fo, with P̅>̅Q̅ (pin 1) obtained as the output.

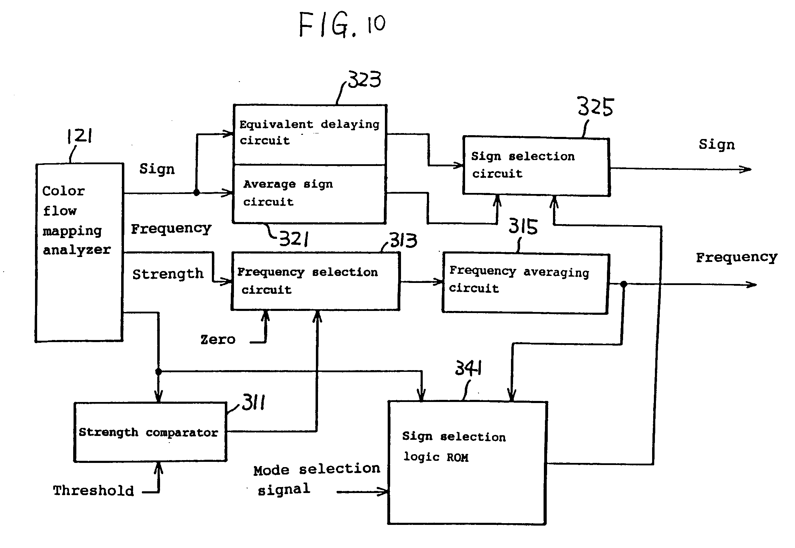

Fig. 9 shows another example configuration of a noise filtering circuit 123. Whilst the configuration shown in Fig. 3 uses the output of frequency averaging circuit 315 and the output of sign selection circuit 325 as the outputs of noise filtering circuit 123, the configuration shown in Fig. 9 uses the output of frequency selection circuit 313 and the output of sign selection circuit 325 as the output of noise filtering circuit 123.

In the configuration shown in Fig. 9 where direct output is obtained from frequency selection circuit 313, output frequency f may contain noise elements. However, strength p corresponds to a threshold greater than given Po, which should provide that no substantial problems arise. Thus, in each of modes 1 to 4, colour indications can be obtained on the basis of selected average sign s̅ and frequency f without any effect, or with reduced effect, of noise, providing clean images. Hence, the risk of false diagnosis in hospitals can be prevented or mitigated.

Fig. 10 shows another example configuration of a noise filtering circuit 123. In the configuration shown in Fig. 10, frequency comparator 317 and mode control circuit 331 in Fig. 3 are replaced by a sign selection logic ROM 341. With the selection information of Table 1 stored in sign selection logic ROM 341, various pieces of data needed to specify modes and select a sign are entered as addresses to have the corresponding selection signals output. For example, assuming that average frequency f̅ generated by frequency averaging circuit 315 consists of 3 bits of data, the 3 bits of data, 1 bit of data corresponding to the result of comparison by strength comparator 311, and 2 bits of data specifying mode selection (total of 6 bits of data) are entered into sign selection logic ROM 341 as addresses to have 1 bit of data specifying average sign s̅ or sign s output. The above sign selection logic ROM 341 may be replaced by another semiconductor memory such as a RAM.

In the above configuration, one of modes 1 to 4 is selected by mode control circuit 331 or sign selection logic 341 which covers all the possible modes. When only one mode is selected fixedly, mode control circuit 331 or sign selection logic ROM 341 may be omitted. In such a case, fixed control in accordance with mode 1 is accomplished, e.g. by entering the output of frequency comparator 317 directly into sign selection circuit 325. Fixed control in accordance with mode 2 is accomplished by entering the output of strength comparator 311 directly into sign selection circuit 325. Fixed control in accordance with mode 3 is accomplished by entering the result of logically ANDing the output of strength comparator 311 and that of frequency comparator 317 into sign selection circuit 325. Fixed control in accordance with mode 4 is accomplished by entering the result of logically ORing the output of strength comparator 311 and that of frequency comparator 317 into sign selection circuit 325.

In the configuration shown in Fig. 10, noise filtering circuit 123 outputs average frequency f̅ output from frequency averaging circuit 315. As shown in Fig. 9, however, frequency f as the output of frequency selection circuit 313 may also be output from the noise filtering circuit.

An embodiment of the present invention provides ultrasonic diagnostic apparatus for displaying a moving body in colour, and particularly provides ultrasonic analysis equipment that yields frequency and sign outputs corresponding to the flow speed and flow direction of blood flow by means of a colour flow mapping analyser. The average of signs analysed by a colour flow mapping analyser is calculated and either the average sign or the sign per se is selected as an analysis result for further employment. The selection conditions considered are whether or not frequency analysed by the colour flow mapping analyser is below a given value and whether or not strength analysed thereby is below a given value. If both or any (either) of the two conditions are met, the average sign is selected. In the other cases, the sign itself is selected. The speed distribution of a moving body is displayed on the basis of the average of selected signs and the frequency analysed by the colour flow mapping analyser, whereby effects of noises contained in analysed signs can be considerably mitigated.

In the ultrasonic diagnostic apparatuses mentioned above as embodying the invention the frequency comparator may be configured of sign selection logic memory storing as data the results of comparing address values with a given threshold for their magnitude, said sign selection logic memory receiving as an address the average frequency calculated by said frequency averaging circuit to produce data for specifying selection conditions for said sign selection circuit.

The sign selection logic memory may be ROM or RAM.

## Claims

1. Ultrasonic diagnostic apparatus, comprising:
a colour flow mapping analyser (121; 211, 213, 215, 217) operable to provide flow analysis factors, comprising a sign s corresponding to the direction of movement of a moving body and a frequency f corresponding to the speed of movement of the moving body, on the basis of received ultrasonic signals reflected from the moving body,
comparator circuitry (123; 311, 317, 341) operable to compare in magnitude one or more flow analysis factors, or quantities derived from the factor or those factors concerned, with a threshold or with respective thresholds, and
means (131) for displaying the speed distribution of the moving body on the basis of the frequency f, or a quantity derived from the frequency f,
characterised in that
the apparatus further comprises:-
an average sign circuit (123; 321) operable to calculate in respect of a given period the average s̅ of signs s provided by the colour flow mapping analyser (121; 211, 213, 215, 217), and
sign selection circuitry (123; 325) arranged to receive the results of comparisons effected in the comparator circuitry (123; 311, 317, 341) and to select either sign s or the average sign s̅ calculated by the average sign circuit (123; 321) for output, in dependence upon the said results,
and in that
the means (131) for displaying the speed distribution of the moving body do so on the basis of the output of the sign selection circuitry (123; 325) and the frequency f, or a quantity derived from the frequency f.

2. Apparatus as claimed in claim 1, further comprising a frequency averaging circuit (315) operable to calculate in respect of a given period the average f̅ of frequencies provided by the colour flow mapping analyser (121; 211, 213, 215, 217).

3. Apparatus as claimed in claim 1 or 2, wherein the colour flow mapping analyser (121; 211, 213, 215, 217) is operable to provide flow analysis factors comprising also strength p of the received ultrasonic signals.

4. Apparatus as claimed in claim 2, wherein the comparator circuitry (317) is operable to compare the average frequency f̅ calculated by the frequency averaging circuit (315) with a first given threshold (Fₒ), and the sign selection circuitry (325) is operable to select sign s for output if the average frequency f̅ is above that first given threshold (Fₒ), and to select the average sign s̅ for output if the average frequency f̅ is below that first given threshold (Fₒ).

5. Apparatus as claimed in claim 3, wherein the comparator circuitry (311) is operable to compare strength p with a second given threshold (Pₒ), and the sign selection circuitry (325) is operable to select sign s for output if the strength p is above that second given threshold (Pₒ), and to select the average sign s̅ for output if the strength p is below that second given threshold (Pₒ).

6. Apparatus as claimed in claims 2 and 3, wherein the comparator circuitry (317, 311) is operable to compare the average frequency f̅ calculated by the frequency averaging circuit (315) with the first given threshold (Fₒ) and to compare strength p with the second given threshold (Pₒ), and the sign selection circuitry (325) is operable to select sign s for output if the average frequency f̅ is above the first given threshold (Fₒ) and the strength p is above the second given threshold (Pₒ), and to select the average sign s̅ for output in other cases.

7. Apparatus as claimed in claims 2 and 3, wherein the comparator circuitry (317, 311) is operable to compare the average frequency f̅ calculated by the frequency averaging circuit (315) with a first given threshold (Fₒ) and to compare strength p with a second given threshold (Pₒ), and the sign selection circuitry (325) is operable to select average sign s̅ for output if the average frequency f̅ is below the first given threshold (Fₒ) and the strength p is below the second given threshold (Pₒ), and to select the sign s for output in other cases.

8. Apparatus as claimed in any one of claims 3 to 7 read as appended to claim 2, wherein the said means (131) for displaying the speed distribution of the moving body do so on the basis of the output of the sign selection circuitry (325) and the average frequency f̅.

9. Apparatus as claimed in claim 4, 6 or 7, or claim 8 when read as appended to claim 4, 6 or 7, wherein the comparator circuitry operable to compare average frequency f̅ with a threshold comprises a sign selection logic memory (341) storing as data the results of comparing address values with the threshold, said sign selection logic memory (341) receiving as an address value the average frequency f̅ to produce data for specifying selection conditions for said sign selection circuit (325).

10. Apparatus as claimed in claim 9, wherein the sign selection logic memory (341) is ROM or RAM.

## Patentansprüche

1. Ultraschalldiagnosevorrichtung, mit:
einem Farbfluß-Mapping-Analysator (121; 211, 213, 215, 217), der betreibbar ist, um Flußanalysefaktoren, die ein Vorzeichen s entsprechend der Bewegungsrichtung eines sich bewegenden Körpers und eine Frequenz f entsprechend der Bewegungsgeschwindigkeit des sich bewegenden Körpers umfassen, auf Basis der vom sich bewegenden Körper reflektierten Ultraschall-Empfangssignale vorzusehen,
einem Komparator-Schaltungsaufbau (123; 311, 317, 341), der betreibbar ist, um in der Größe einen oder mehrere Flußanalysefaktoren, oder von dem betreffenden Faktor oder den betreffenden Faktoren abgeleitete Mengen, mit einer Schwelle oder mit entsprechenden Schwellen zu vergleichen, und
einer Einrichtung (131) für eine Anzeige der Geschwindigkeitsverteilung des sich bewegenden Körpers auf Basis der Frequenz f, oder einer von der Frequenz f abgeleiteten Menge,
dadurch gekennzeichnet, daß die Vorrichtung ferner umfaßt:
eine Vorzeichen-Mittelwert-Schaltung (123; 321), die betreibbar ist, um in bezug auf eine gegebene Periode den Mittelwert s̅ von Vorzeichen s, die vom Farbfluß-Mapping-Analysator (121; 211, 213, 215, 217) vorgesehen werden, zu berechnen, und
einen Vorzeichen-Auswahlschaltungsaufbau (123; 325), der eingerichtet ist, um die Ergebnisse von im Komparator-Schaltungsaufbau (123; 311, 317, 341) durchgeführten Vergleichen zu empfangen, und entweder das Vorzeichen s oder das von der Vorzeichen-Mittelwert-Schaltung (123; 321) berechnete mittlere Vorzeichen s̅ für einen Ausgang, in Abhängigkeit von den genannten Ergebnissen, auszuwählen,
und daß
die Einrichtung (131) für eine Anzeige der Geschwindigkeitsverteilung des sich bewegenden Körpers dies auf Basis des Ausgangs des Vorzeichen-Auswahlschaltungsaufbaus (123; 325) und der Frequenz f, oder einer von der Frequenz f abgeleiteten Menge, durchführt.

2. Vorrichtung nach Anspruch 1, ferner mit einer Frequenz-Mittelwertbildungsschaltung (315), die betreibbar ist, um in bezug auf eine gegebene Periode den Mittelwert f̅ von Frequenzen, die vom Farbfluß-Mapping-Analysator (121; 211, 213, 215, 217) vorgesehen werden, zu berechnen.

3. Vorrichtung nach Anspruch 1 oder 2, bei welcher der Farbfluß-Mapping-Analysator (121; 211, 213, 215, 217) betreibbar ist, um Flußanalysefaktoren, die auch die Stärke p der Ultraschall-Empfangssignale umfassen, vorzusehen.

4. Vorrichtung nach Anspruch 2, bei welcher der Komparator-Schaltungsaufbau (317) betreibbar ist, um die mittlere Frequenz f̅, die von der Frequenz-Mittelwertbildungsschaltung (315) berechnet wird, mit einer ersten gegebenen Schwelle (Fo) zu vergleichen, und der Vorzeichen-Auswahlschaltungsaufbau (325) betreibbar ist, um das Vorzeichen s für einen Ausgang auszuwählen, wenn die mittlere Frequenz f̅ über jener ersten gegebenen Schwelle (Fo) liegt, und um das mittlere Vorzeichen s̅ für einen Ausgang auszuwählen, wenn die mittlere Frequenz f̅ unter jener ersten gegebenen Schwelle (Fo) liegt.

5. Vorrichtung nach Anspruch 3, bei welcher der Komparator-Schaltungsaufbau (311) betreibbar ist, um die Stärke p mit einer zweiten gegebenen Schwelle (Po) zu vergleichen, und der Vorzeichen-Auswahlschaltungsaufbau (325) betreibbar ist, um das Vorzeichen s für einen Ausgang auszuwählen, wenn die Stärke p über jener zweiten gegebenen Schwelle (Po) liegt, und um das mittlere Vorzeichen s̅ für einen Ausgang auszuwählen, wenn die Stärke p unter jener zweiten gegebenen Schwelle (Po) liegt.

6. Vorrichtung nach Anspruch 2 oder 3, bei welcher der Komparator-Schaltungsaufbau (317, 311) betreibbar ist, um die mittlere Frequenz f̅, die von der Frequenz-Mittelwertbildungsschaltung (315) berechnet wird, mit der ersten gegebenen Schwelle (Fo) zu vergleichen, und um die Stärke p mit der zweiten gegebenen Schwelle (Po) zu vergleichen, und der Vorzeichen-Auswahlschaltungsaufbau (325) betreibbar ist, um das Vorzeichen s für einen Ausgang auszuwählen, wenn die mittlere Frequenz f̅ über der ersten gegebenen Schwelle (Fo) liegt, und die Stärke p über der zweiten gegebenen Schwelle (Po) liegt, und um in anderen Fällen das mittlere Vorzeichen s̅ für einen Ausgang auszuwählen.

7. Vorrichtung nach Anspruch 2 oder 3, bei welcher der Komparator-Schaltungsaufbau (317, 311) betreibbar ist, um die mittlere Frequenz f̅, die von der Frequenz-Mittelwertbildungsschaltung (315) berechnet wird, mit der ersten gegebenen Schwelle (Fo) zu vergleichen, und um die Stärke p mit der zweiten gegebenen Schwelle (Po) zu vergleichen, und der Vorzeichen-Auswahlschaltungsaufbau (325) betreibbar ist, um das mittlere Vorzeichen s̅ für einen Ausgang auszuwählen, wenn die mittlere Frequenz f̅ unter der ersten gegebenen Schwelle (Fo) liegt, und die Stärke p unter der zweiten gegebenen Schwelle (Po) liegt, und um in anderen Fällen das Vorzeichen s für einen Ausgang auszuwählen.

8. Vorrichtung nach einem der Ansprüche 3 bis 7, wenn auf Anspruch 2 rückbezogen, bei welcher die genannte Einrichtung (131) für eine Anzeige der Geschwindigkeitsverteilung des sich bewegenden Körpers dies auf Basis des Ausgangs des Vorzeichen-Auswahlschaltungsaufbaus (325) und der mittleren Frequenz f̅ durchführt.

9. Vorrichtung nach Anspruch 4, 6 oder 7, oder 8, wenn auf Anspruch 4, 6 oder 7 rückbezogen, bei welcher der Komparator-Schaltungsaufbau, der betreibbar ist, um die mittlere Frequenz f̅ mit einer Schwelle zu vergleichen, einen Vorzeichenauswahl-Logikspeicher (341), der die Ergebnisse eines Vergleichs von Adressenwerten mit der Schwelle als Daten speichert, umfaßt, wobei der genannte Vorzeichenauswahl-Logikspeicher (341) die mittlere Frequenz f̅ als Adressenwert empfängt, um Daten zum Spezifizieren von Auswahlbedingungen für die genannte Vorzeichen-Auswahlschaltung (325) zu erzeugen.

10. Vorrichtung nach Anspruch 9, bei welcher der Vorzeichenauswahl-Logikspeicher (341) ein ROM oder RAM ist.

## Revendications

1. Appareil de diagnostic à ultrasons comprenant :
un analyseur de cartographie d'écoulement en couleurs (121 ; 211, 213, 215, 217) servant à fournir des facteurs d'analyse d'écoulement, comprenant un signe s correspondant au sens de déplacement d'un corps en mouvement et une fréquence f correspondant à la vitesse de déplacement du corps en mouvement, sur la base de signaux ultrasonores reçus, réfléchis par le corps en mouvement ;
des circuits comparateurs (123 ; 311, 317, 341) servant à comparer en amplitude un ou plusieurs des facteurs d'analyse d'écoulement, ou des grandeurs obtenues à partir du facteur ou des facteurs concerné(s), à un seuil ou à des seuils respectif(s) ; et,
un moyen (131) pour afficher la répartition de vitesse du corps en mouvement sur la base de la fréquence f, ou d'une grandeur dérivée de la fréquence f ;
caractérisé en ce que :
l'appareil comprend en outre :
un circuit de signe moyen (123 ; 321) servant à calculer, en ce qui concerne une période donnée, la moyenne s̅ des signes s fournis par l'analyseur de cartographie d'écoulement en couleurs (121 ; 211, 213, 215, 217) ; et,
des circuits de sélection de signe (123 ; 325) disposés pour recevoir les résultats des comparaisons effectuées dans les circuits comparateurs (123 ; 311, 317, 341) et pour sélectionner, comme sortie en fonction desdits résultats, l'un ou l'autre du signe s ou du signe moyen s̅ calculé par le circuit de signe moyen (123 ; 321) ; et,
en ce que :
le moyen d'affichage (131) pour afficher la répartition de vitesse du corps en mouvement le fait sur la base de la sortie des circuits de sélection de signe (123 ; 325) et de la fréquence f, ou de la grandeur dérivée de la fréquence f.

2. Appareil selon la revendication 1, comprenant en outre un circuit de calcul de moyenne de fréquence (315) servant à calculer, en ce qui concerne une période donnée, la moyenne f̅ des fréquences fournies par l'analyseur de cartographie d'écoulement en couleurs (121 ; 211, 213, 215, 217).

3. Appareil selon la revendication 1 ou 2, dans lequel l'analyseur de cartographie d'écoulement en couleurs (121 ; 211, 213, 215, 217) sert à fournir des facteurs d'analyse d'écoulement comprenant également l'intensité p des signaux ultrasonores reçus.

4. Appareil selon la revendication 2, dans lequel le circuit comparateur (317) sert à comparer la fréquence moyenne f̅ calculée par le circuit de calcul de moyenne de fréquence (315) à un premier seuil (Fo) donné, et dans lequel les circuits de sélection de signe (325) servent à sélectionner le signe s comme sortie si la fréquence moyenne f̅ est au-dessus de ce premier seuil (Fo) donné, et à sélectionner le signe moyen s comme sortie si la fréquence moyenne f̅ est inférieure à ce premier seuil (Fo) donné.

5. Appareil selon la revendication 3, dans lequel le circuit comparateur (311) sert à comparer l'intensité p à un second seuil (Po) donné, et dans lequel les circuits de sélection de signe (325) servent à sélectionner le signe s comme sortie si l'intensité p est au-dessus de ce second seuil (Po) donné, et à sélectionner le signe moyen s̅ comme sortie si l'intensité p est inférieure à ce second seuil (Po) donné.

6. Appareil selon les revendications 2 et 3, dans lequel le circuit comparateur (317, 311) sert à comparer la fréquence moyenne f̅ calculée par le circuit de calcul de moyenne de fréquence (315) au premier seuil (Fo) donné, et à comparer l'intensité p au second seuil (Po) donné, et dans lequel les circuits de sélection de signe (325) servent à sélectionner le signe s comme sortie si la fréquence moyenne f̅ est au-dessus du premier seuil (Fo) donné et si l'intensité p est au-dessus du second seuil (Po) donné, et à sélectionner le signe moyen s̅ comme sortie dans les autres cas.

7. Appareil selon les revendications 2 et 3, dans lequel le circuit comparateur (317, 311) sert à comparer la fréquence moyenne f̅ calculée par le circuit de calcul de moyenne de fréquence (315) au premier seuil (Fo) donné, et à comparer l'intensité p au second seuil (Po) donné, et dans lequel les circuits de sélection de signe (325) servent à sélectionner le signe moyen s̅ comme sortie si la fréquence moyenne f̅ est au-dessous du premier seuil (Fo) donné et si l'intensité p est au-dessous du second seuil (Po) donné, et à sélectionner le signe s comme sortie dans les autres cas.

8. Appareil selon l'une quelconque des revendications 3 à 7, lorsqu'elle dépend de la revendication 2, dans lequel ledit moyen (131) pour afficher la répartition de vitesse du corps en mouvement le fait sur la base de la sortie des circuits de sélection de signe (325) et de la fréquence moyenne f̅.

9. Appareil selon la revendication 4, 6 ou 7, ou la revendication 8 lorsqu'elle dépend de la revendication 4, 6 ou 7, dans lequel les circuits comparateurs qui servent à comparer la fréquence moyenne f̅ à un seuil, comprennent une mémoire logique de sélection de signe (341) mémorisant comme données les résultats de la comparaison de valeurs d'adresse au seuil, ladite mémoire logique de sélection de signe (341) recevant comme valeur d'adresse la fréquence moyenne f̅ pour produire une donnée pour spécifier les conditions de sélection par ledit circuit de sélection de signe (325).

10. Appareil selon la revendication 9, dans lequel la mémoire logique de sélection de signe (341) est une ROM ou une RAM.
